## Europäisches Patentamt
## European Patent Office
## Office européen des brevets

(19)

(11) Veröffentlichungsnummer: **0 002 460**
**B1**

(12) **EUROPÄISCHE PATENTSCHRIFT**

(21) Anmeldenummer: **78101452.7**

(22) Anmeldetag: **24.11.78**

(51) Int. Cl.³: **C 07 D 317/60**

(54) Verfahren zur Herstellung von 3,4-Methylendioxymandelsäure

(30) Priorität: **07.12.77 DE 2754490**

(43) Veröffentlichungstag der Anmeldung:
**27.06.79 Patentblatt 79/13**

(45) Bekanntmachung des Hinweises auf die Patenterteilung:
**25.06.80 Patentblatt 80/13**

(84) Benannte Vertragsstaaten:
**CH DE FR GB NL**

(73) Patentinhaber: **Haarmann & Reimer GMBH**
**Postfach 138, D - 3450 Holzminden (DE)**

(72) Erfinder: **Bauer, Kurt, Dr.**
**Corveyblick 41**
**D - 3450 Holzminden (DE)**
**Mölleken, Reiner, Dr.**
**Forstbachtal 35**
**D - 3451 Golmbach Ortsteil Warbsen (DE)**

(74) Vertreter: **Dill, Erwin, Dr.**
**c/o BAYER AG Zentralbereich Patente Marken**
**und Lizenzen Bayerwerk**
**D - 5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 002 460 B1

Verfahren zur Herstellung von 3,4-Methylendioxymandelsäure

Die Erfindung betrifft ein neues Verfahren zur Herstellung von 3,4-Methylendioxymandelsäure.

3,4 Methylendioxymandelsäure wurde bislang in Analogie zu dem üblichen Herstellungsverfahren für Mandelsäure und Mandelsäurederivate aus Piperonal über das Piperonalcyanhydrin hergestellt (Chemisches Zentralblatt *1909*, Seiten 1927—28). Dieses verfahren ist jedoch wegen des erforderlichen teuren Ausgangsproduktes Piperonal technisch uninteressant.

Es wurde nun gefunden, daß man 3,4-Methylendioxymandelsäure, ausgehend von den billigeren, leicht zugänglichen Ausgangsmaterialien 1,2-Methylendioxybenzol und Glyoxylsäure auf einfache Weise und in ausgezeichneten Ausbeuten herstellen kann, wenn man 1,2-Methylendioxybenzol mit Glyoxylsäure in stark saurem Medium umsetzt.

Die Säurestärke der Glyoxylsäure, wenn sie in Form des Mono- oder Semihydrates eingesetzt wird, ist an sich ausreichend zur Katalysierung der Reaktion. Wegen der zur Umsetzung empfehlenswerten Reaktionstemperaturen von etwa 60—80°C entstehen jedoch erhebliche Mengen an Bis-(3,4-methylendioxyphenyl)-essigsäure. Dieses Nebenprodukt ent steht durch Anlagerung eines weiteren 1,2-Methylendioxy - benzol - Moleküls an die gewünschte 3,4-Methylendioxymandelsäure.

Es hat sich daher als vorteilhaft erwiesen, als Katalysatoren stärkere Säuren als Glyoxylsäure, die sich unter den Reaktionsbedingungen inert verhalten, z. B. starke Mineralsäuren wie 85 %ige Phosphorsäure, 70 %ige Schwefelsäure, konzentrierte oder gasförmige Chlorwasserstoffsäure, oder starke organische Säuren, wie Trifluoressigsäure und Dichloressigsäure zuzusetzen. Durch Zusatz dieser Säuren kann die Reaktionstemperatur auf O bis 60°C gesenkt und dadurch die Bildung von Bis-(3,4-methylendioxyphenyl) - essigsäure stark her-. abgesetzt werden. Unter stärkeren Säuren als Glyoxylsäure werden im Rahmen des erfindungsgemäßen Verfahrens solche Säuren Verstanden, deren Dissoziationskonstante in Wasser über $4,74.10^{-4}$ (Dissoziationskonstante der Glyoxylsäure), vorzugsweise über $10^{-3}$ beträgt.

Wird die Glyoxylsäure in Form wäßriger Lösungen, z.B. der handelsüblichen 50 %igen wäßrigen Lösung, eingesetzt, so ist es zur Erzielung der notwendigen Säurestärke erforderlich, eine größere Menge der starken Mineral- und organischen Säuren oder, falls die Säuren selbst Wasser enthalten sollten, Säuren höherer Konzentration einzusetzen, um den Verdünnungseffekt des Wassers auszugleichen.

Da die Ausgangsmaterialien häufig nicht miteinander mischbar, bzw. ineinander löslich sind und das Reaktionsproduct aufgrund seiner geringen Löslichkeit überwiegend kristallin anfällt, ist es vorteilhaft, das heterogene Reaktionsgemisch zu rühren.

Zur Erhaltung der Rührfähigkeit kann es vorteilhaft sein, wesentlich größere Mengen der starken Säure einzusetzen, als zur Erzielung der Säurestärke notwendig ist. Der Nachteil, daß in dieser größeren Säuremenge nach dem Abfiltrieren des Reaktionsproduktes auch ein größerer Teil des Reaktionsproduktes gelöst zurückbleibt, kann dadurch ausgeglichen werden, daß man die Säure, gegebenenfalls nach dem Aufkonzentrieren, wieder einsetzt.

Die Reaktion wird bevorzugt ohne Lösungsmittel durchgeführt. Der Zusatz eines unter den Reaktionsbedingungen stabilen, vorzugsweise polaren Lösungsmittels, z.B. Ameisensäure oder Essigsäure, kann sich jedoch in manchen Fällen vorteilhaft auf den Reaktionsverlauf auswirken, solange die für die Katalyse der Reaktion notwendige Säurestärke erhalten bleibt, da das Lösungsmittel als Lösungsvermittler zwischen den verschiedenen Phasen wirkt. Besonders geeignete Lösungsmittel sind die gleichzeitig als starke Säure wirkenden organischen Säuren wie Trifluoressigsäure und Dichloressigsäure.

Die Ausgangsverbindungen 1,2-Methylendioxybenzol und Glyoxylsäure werden bevorzugt in äquimolaren Mengen eingesetzt.

Der Verlauf der erfindungsgemäßen Reaktion ist überrachend, da aus Gazz. Chim. Ital. *96* (4), 465 (1966) bekannt ist, daß zwar die Umsetzung von Glyoxylsäurealkylestern mit alkylsubstituierten Aromaten in stark saurem Medium zu Mandelsäurederivaten führt, mit alkoxysubstituierten Aromaten wie Veratrol jedoch nur Diphenylessigsäurederivate liefert.

3,4-Methylendioxymandelsäure ist u.a. ein wichtiges Ausgangsmaterial für die Herstellung des wertvollen Riechstoffs Piperonal. Piperonal wird aus 3,4-Methylendioxymandelsäure durch oxydative Decarboxylierung hergestellt (siehe Current Sci. (India) *27*, 22 (1958).

*Beispiel 1*

46 g (0,5 Mol) Glyoxylsäuremonohydrat, 61 g (0,5 Mol) 1,2-Methylendioxybenzol und 200 g (1,74 Mol) 85 %ige Phosphorsäure werden dei 25° C zusammengegeben und 5 Stunden gerührt. Dabei steigt die Temperatur auf 38° C. Zur Erhaltung der Rührfähigkeit des Reaktionsgemisches werden 40 ml Wasser zugesetzt. Nach 5 Stunden werden weitere 160 ml Wasser zugesetst 10 Minuten nachgerührt und das anfallende Kristallisat abgesaugt. Das Kristallisat wird bei 85° C in 650 ml Wasser und 100 ml Toluol gelöst und die Phasen getrennt. Aus der Toluolphase werden nach mehrmaligem Waschen mit 20 %iger Natronlauge und Abdestillieren des Lösungsmittels 7,1 g 1,2-Methylendioxybenzol zurückgewonnen. Aus der wässrigen Phase kristallisieren nach dem Abkühlen auf Raumtemperatur 57,6 g und nach dem Einengen der Mutterlauge weitere 8,3 g 3,4-Methylendioxymandelsäure aus. Die Ausbeute an 3,4-Methylendioxymandelsäure beträgt 76,1 % der Theorie, bezogen auf umgesetztes 1,2-Methylendioxybenzol. Sie kann durch Aufarbeiten des stark sauren Filtrats des Reaktionsgemisches noch erhöht werden. Da die Aufarbeitung aber sehr aufwendig ist, ist es vorteilhafter, das saure Filtrat aufzukonzentrieren und erneut einzusetzen.

*Beispiel 2*

Zu einer Mischung von 148 g (1 Mol) 50 %iger wässriger Glyoxylsäure und 122 g (1 Mol) 1,2-Methylendioxybenzol werden innerhalb von 40 Minuten bei 5° C unter Rühren 200 g 90 %ige Schwefelsäure getropft. Nach weiterem 6-stündigem Rühren werden 500 g Eiswasser zugesetzt und 10 Minuten nachgerührt. Die ausgefallenen Kristalle werden abgesaugt und wie in Beispiel 1 aufgearbeitet.

Man gewinnt aus der Toluolphase 6,1 g 1,2-Methylendioxybenzol zurück. Aus der wäßrigen Phase erhält man 167,3 g 3,4-Methylendioxymandelsäure, entsprechend einer Ausbeute von 85,3 % der Theorie, bezogen auf umgesetztes 1,2-Methylendioxybenzol.

*Beispiel 3*

Eine Mischung aus 92 g (1 mol) Glyoxylsäuremonohydrat und 122 g (1 Mol) Methylendioxybenzol wird bei 80° C 4 Stunden gerührt, danach mit 700 ml Wasser und 150 ml Toluol versetzt und die Phasen getrennt. Aus der wässrigen Phase kristallisieren nach dem Abkühlen auf Raumtemperatur 44,7 g und nach dem Einengen der wässrigen Phase weitere 17,3 g 3,4-Methylendioxymandelsäure aus. Die Toluolphase ergibt nach dem Ausschütteln mit 100 ml 20 %iger Natronlauge und Abdestillieren des Toluols 10,5 g nicht umgesetztes 1,2-Methylenedioxybenzol. Die Ausbeute an 3,4-Methylendioxymandelsäure beträgt 34,6 % der Theorie bezogen auf umgesetztes 1,2-Methylendioxybenzol.

*Beispiel 4*

39 g (o,424 Mol) Glyoxylsäuremonohydrat werden in 126 g Trifluoressigsäure gelöst und bei 20° C mit 51,7 g (0,424 Mol) 1,2-Methylendioxybenzol versetzt. Die zunächst homogene Mischung wird 3,5 Stunden bei 20° C gerührt. Dann werden die ausgefallenen Kristalle abgesaugt und wie in Beispiel 1 beschrieben aufgearbeitet. Aus der Toluolphase werden 10 g Bis- (3,4-methylendioxyphenyl)-essigsäure erhalten. Aus der wäßrigen Phase erhält man 62,2 g 3,4 Methylendioxymandelsäure. Das entspricht einer Ausbeute von 75 % der Theorie, bezogen auf eingesetztes 1,2-Methylendioxybenzol. Die Ausbeute kann durch Aufarbeitung des stark sauren Filtrats des Reaktionsgemisches erhöht werden. Vorteilhafter ist es jedoch, das saure Filtrat aufzukonzentrieren und erneut einzusetzen.

Setzt man anstelle von Glyoxylsäuremonohydrat eine äquivalente Menge 50 %iger wäßriger Glyoxylsäure ein und rührt anstatt 3,5 Stunden 6 Stunden, dann werden aus der Toluolphase 8,58 g Bis-3,4-methylendioxyphenylessigsäure und aus der wäßrigen Phase 73,4 g 3,4-Methylendioxymandelsäure erhalten, entsprechend einer Ausbeute von 76,3 % der Theorie, bezogen auf eingesetztes 1,2-Methylendioxybenzol.

## Patentansprüche

1. Verfahren zur Herstellung von 3,4-Methylendioxymandelsäure, dadurch gekennzeichnet, daß man Glyoxylsäure mit 1,2-Methylendioxybenzol in stark saurem Medium umsetzt.

2. Verfahren nach Anspruch 1, dadurch gekennzeichnet, daß die Umsetzung in Gegenwart stärkerer Säuren als Glyoxylsäure durch geführt wird.

3. Verfahren nach Anspruch 1 und 2, dadurch gekennzeichnet, daß die Umsetzung in Gegenwart von Phosphorsäure, Schwefelsäure, gasförmiger oder konzentrierter Chlorwasserstoffsäure, Trifluoressigsäure oder Dichloressigsäure durchgeführt wird.

## Revendications

1. Procédé de production d'acide 3,4-méthylènedioxymandélique, caractérisé en ce qu'il consiste à faire réagir de l'acide glyoxylique avec du 1,2-méthylènedioxybenzène en milieu fortement acide.

2. Procédé suivant la revendication 1, caractérisé en ce que la réaction est conduite en présence d'acides plus forts que l'acide glyoxylique.

3. Procédé suivant l'une des revendications 1 et 2, caractérisé en ce que la réaction est conduite en présence d'acide phosphorique, d'acide sulfurique, de gaz chlorhydrique ou d'acide chlorhydrique concentré, d'acide trifluoracétique ou d'acide dichloracétique.

## Claims

1. Process for the preparation of 3,4-methylenedioxy-mandelic acid, characterised in that glyoxylic acid is reacted with 1,2-methylenedioxybenzene in a strongly acid medium.

2. Process according to Claim 1, characterised in that the reaction is carried out in the presence of stronger acids than glyoxylic acid.

3. Process according to Claim 1 and 2, characterised in that the reaction is carried out in the presence of phosphoric acid, sulphuric acid, gaseous or concentrated hydrochloric acid, trifluoroacetic acid or dichloroacetic acid.